Europäisches Patentamt

European Patent Office

Office européen des brevets

(19)

(11) Numéro de publication : **0 287 426 B1**

## FASCICULE DE BREVET EUROPEEN

(12)

(45) Date de publication du fascicule du brevet :
24.07.91 Bulletin 91/30

(51) Int. Cl.⁵ : **C07C 69/68, C07C 67/08**

(21) Numéro de dépôt : **88400797.2**

(22) Date de dépôt : **01.04.88**

(54) Procédé de préparation d'esters de l'acide lactique optiquement purs.

(30) Priorité : **13.04.87 FR 8705407**

(43) Date de publication de la demande :
**19.10.88 Bulletin 88/42**

(45) Mention de la délivrance du brevet :
**24.07.91 Bulletin 91/30**

(84) Etats contractants désignés :
**AT BE CH DE ES FR GB GR IT LI LU NL SE**

(56) Documents cités :
**US-A- 2 406 648
US-A- 2 410 740
US-A- 2 519 906**

(73) Titulaire : **RHONE-POULENC CHIMIE
25, quai Paul Doumer
F-92408 Courbevoie Cédex (FR)**

(72) Inventeur : **Alas, Michel
12, rue Eloi Ricard
F-79500-Melle (FR)**
Inventeur : **Bouniot, Albert
Bois Joli Paizay Le Tort
F-79500-Melle (FR)**
Inventeur : **Redien, Pierre
Perrière de Vhail
F-79500-Melle (FR)**

(74) Mandataire : **Le Pennec, Magali et al
RHONE-POULENC INTERSERVICES Service
Brevets Chimie 25, Quai Paul Doumer
F-92408 Courbevoie Cédex (FR)**

EP 0 287 426 B1

## Description

La présente invention concerne un procédé de préparation d'esters de l'acide lactique. Elle concerne plus particulièrement un procédé amélioré d'estérification de l'acide lactique par des alcools contenant 1 à 12 atomes de carbone.

Les procédés classiques de préparation de lactate de méthyle consistent à condenser l'acide lactique avec le méthanol en présence d'un acide minéral tel que l'acide borique, phosphorique et tout particulièrement l'acide sulfurique. L'acide sulfurique utilisé comme catalyseur d'estérification doit ensuite être séparé du lactate de méthyle par neutralisation au carbonate de sodium suivi d'une séparation par filtration du sulfate de sodium produit. Le rendement obtenu en fin d'opération sur l'acide lactique introduit ne dépasse généralement pas 80%.

L'industrie a cherché à modifier l'agent de neutralisation de façon à éviter la formation de sulfate de sodium qui a tendance à boucher les colonnes mais aucun des agents de neutralisation essayés (soude, potasse) n'a permis d'atteindre un procédé satisfaisant industriellement.

Il est aussi connu d'après le brevet anglais n° 907322 de réaliser l'estérification à une température d'environ 150°C de façon à provoquer la vaporisation du lactate de méthyle qui est entraîné en tête de la colonne d'estérification en même temps que l'alcool.

Dans les conditions d'estérification décrites dans le brevet GB 907322 c'est à dire aux environs de 150°C on observe une racémisation du motif lactique d'autant plus importante que la température est élevée. D'autre part l'ester sort en tête de colonne mélangé à l'alcool et à l'eau de réaction d'où il faut le séparer.

Il a maintenant été trouvé un nouveau procédé de préparation de lactate de méthyle présentant un degré de pureté en un seul enantiomère surprenant et un rendement chimique calculé sur l'acide lactique néanmoins toujours excellent (supérieur à 98%).

Ce procédé consiste à effectuer une estérification de l'acide lactique optiquement actif par un alcool contenant 1 à 12 atomes de carbone dans une enceinte, à une température inférieure à 140°C et en l'absence de catalyseur.

L'avantage de ce procédé par rapport au brevet GB 907 322 consiste à effectuer l'estérification sans distiller l'ester en tête de colonne, ce qui évite les problèmes de bouchage dûs aux impuretés de l'acide lactique telles que les ions minéraux : sodium, magnésium, potassium, calcium, les acides amines et les protéines diverses. En effet les impuretés diverses sont solubles dans la solution formée par l'ester et l'excès d'alcool alors que dans le cas du brevet GB 907 322 où l'ester et l'alcool sont éliminés en tête de colonne les impuretés précipitent et obturent rapidement la colonne, d'après nos propres expériences nous avons constaté qu'en 5 jours il est impossible de faire passer tout le liquide dans la colonne car elle est totalement colmatée.

Le problème avait été circonscrit par HOECHST dans son brevet DE 3.222.837 par l'addition d'un solvant lourd (propanediol et éthylène oxyde de poids moléculaire compris entre 400 et 2000) qui permet de solubiliser les impuretés de l'acide lactique et les sous produits lourds issus de l'estérification. Ce procédé résoud le problème de colmatage mais à un coût qui ne le rend pas industrialisable.

L'autre avantage du procédé de la présente invention par rapport au brevet GB 907 322 consiste à effectuer l'estérification à une température inférieure ce qui minimise considérablement les problèmes de racémisation.

Il permet en outre une estérification avec des alcools lourds contenant plus de 2 atomes de carbone, ce que ne permettait pas le procédé du brevet anglais précédemment mentionné.

Les alcools utilisés dans le procédé de la présente invention dont des alcools primaires ou secondaires aliphatiques linéaires ramifiés ou cycliques contenant 1 à 12 atomes de carbone. Les diols peuvent être aussi utilisés dans le procédé de l'invention. On peut citer non limitativement le méthanol, l'éthanol, l'isopropanol, l'isobutanol, l'éthyl-2 hexanol, l'éthylène glycol, le cyclohexanol.

Lorsque les alcools utilisés sont des alcools aliphatiques contenant plus de quatre atomes de carbone, on ajoute avantageusement lors de la séparation de l'alcool du milieu réactionnel par distillation, un agent formant un azéotrope avec l'eau produite par l'estérification tel que par exemple le toluène, le benzène, le xylène, l'oxyde d'isopropyle, l'oxyde de butyle, le méthyltertiobutyléther, les hydrocarbures éventuellement chlorés, le cyclohexane.

Pour une meilleure mise en oeuvre de l'invention, on préfère utiliser un rapport molaire de l'alcool à l'acide lactique compris entre 1,3 et 4. Dans le cas des alcools lourds contenant plus de 4 atomes de carbone le rapport sera proche de 1,3 et dans le cas des alcools les plus les légers il sera voisin de 4.

Il est évident que l'alcool en excès sera séparé et recyclé pour une nouvelle estérification.

La quantité d'agent formant un azéotrope avec l'eau sera calculée par tout homme du métier de façon à éliminer toute l'eau formée au cours de l'estérification. Cette quantité variera donc en fonction de l'agent choisi et de la quantité d'acide lactique intoduite.

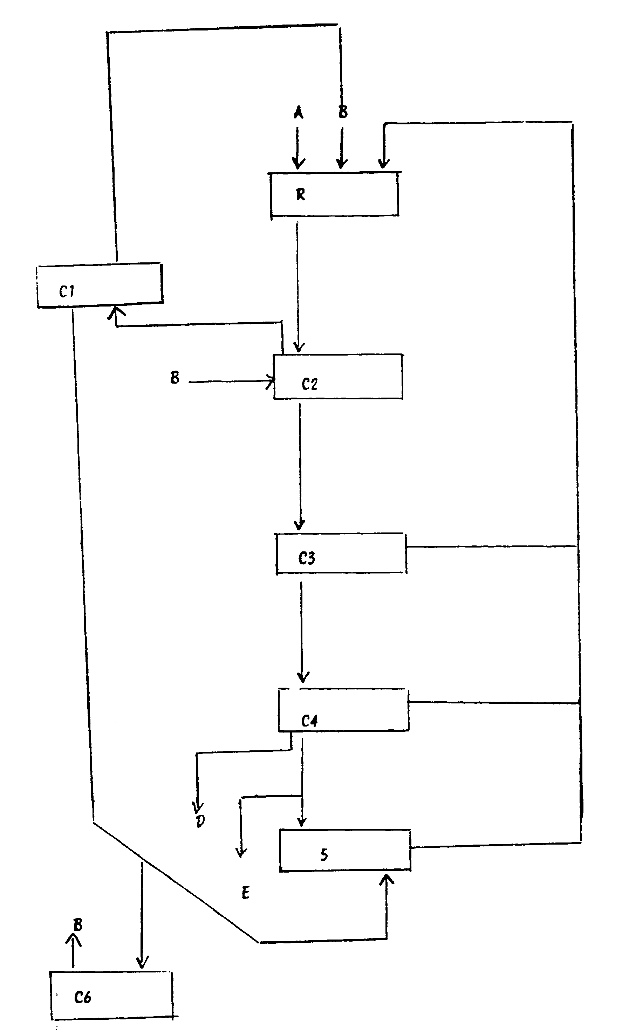

L'invention va être décrite plus complétement afin d'en détailler le fonctionnement conformément au dessin ci-après annexé.

Dans un réacteur ou une colonne d'estérification référencé R on introduit l'alcool (B) et l'acide lactique (A) optiquement actif. On chauffe le réacteur entre 90 et 140°C (de préférence entre 95 et 130°C) pendant 5 à 12 heures et de préférence pendant environ 8 heures. L'alcool bouillant assure le mélange du milieu réactionnel.

Après la période d'estérification le mélange obtenu est introduit dans un colonne "à plateaux profonds" $(C_2)$ où l'on introduit aussi un excès d'alcool surchauffé (B) lorsque ce dernier contient au maximum 4 atomes de carbone ou un agent formant un azéotrope avec l'eau lorsque l'alcool contient plus de 4 atomes de carbone. On entend par "colonne" à plateaux profonds" toute colonne permettant un contact prolongé entre deux liquides ou un gaz et un liquide introduits à contre courant l'un de l'autre.

En tête de la colonne $C_1$ surmontant la colonne $C_2$ on élimine un mélange eau-alcool ou un mélange eau-alcool-azéotrope lorsque l'on utilise un tel agent.

Il est évident que pour la compréhension de l'invention, le réacteur ou la colonne R et les colonnes $C_1$ et $C_2$ ont été différenciés, mais l'invention ne doit pas être réduite à ce type d'installation ; elle couvre tout type équivalent d'installation permettant un contact même dans une seule unité d'un alcool et d'acide lactique et l'élimination de l'alcool excédentaire et de l'eau formée au cours de l'estérification.

L'eau et l'alcool ou l'eau, l'alcool et l'agent formant un azéotrope avec l'eau sont séparés par toute toute méthode classique connue de l'homme de l'art dans une colonne $C_6$. L'alcool pur et anhydre sortant de la colonne $C_6$ est réintroduit dans l'enceinte R.

L'ester lactique brut récupéré au pied de la colonne $C_2$ contient une quantité importante de polylactates, d'acides polylactiques de sels minéraux et de protéines. Il est introduit dans une colonne $C_3$ afin d'éliminer tout l'alcool résiduaire et les traces éventuelles d'agent formant un azéotrope sous pression réduite.

Le résidu après distillation est introduit dans un colonne $(C_4)$ afin de séparer l'ester (D) par distillation sous pression réduite comprise entre environ $10^4$ et $10^2$ Pascal.

Après distillation de l'ester les sels minéraux précipitent et sont éliminés par simple décantation dans une enceinte E.

Les polylactates résiduaires sont dépolymérisés par un traitement à l'eau et/ou à l'alcool dans une enceinte 5 à une température comprise entre 100 et 160°C et de préférence d'environ 130°C.

L'eau, l'alcool ou le mélange eau-alcool servant d'agent de dépolymérisation contient de préférence 0 à 20% en poids d'alcool pour les alcools lourds contenant plus de quatre atomes de carbone de façon à conserver une phase homogène. Ce mélange eau-alcool peut avantageusement provenir de la colonne $C_1$.

Ce traitement de polylactates dure avantageusement 1 à 3 heures.

La quantité de mélange eau-alcool rapportée à la quantité de polylactates à dépolymériser est avantageusement comprise entre 10 et 35% en poids.

Après cette étape de traitement des polylactates on obtient un mélange acide lactique-alcool contenant une très faible quantité de produits faiblement polymérisés qui est recyclée dans l'enceinte R.

L'invention va être plus complètement décrite à l'aide des exemples suivants qui ne doivent pas être considérés comme limitatifs de l'invention. Elle comprend tous les moyens constituant des équivalents techniques des moyens décrits.

## EXEMPLE 1

Dans une cuve estérificatrice chauffée ayant un volume de 100 cm³ et surmontée d'une colonne de distillation débordant dans une colonne à plateaux profonds, on alimente en continu 20 g/h d'acide lactique hydraté à 80% d'acide lactique et 20 g/h de méthanol en chauffant pour tenir la température à 95-130°. On souffle à la base de la colonne à plateaux profonds 30 g/h de méthanol que l'on vaporise au préalable à 120-150°C.

On récupère en tête de colonne un mélange de méthanol et d'eau que l'on régénère à l'état de méthanol sec par distillation. En pied de la colonne à plateaux profonds, on obtient un mélange méthanol, lactate, acide lactique et polymères lactiques (débit 40 g/h). Ce mélange est rectifié sous pression réduite, ce qui permet de récupérer le lactate de méthyle (E = 72°C/6600 Pa (50 Torr) (débit 18 g/h). Les polymères lactiques et l'acide lactique (débit 12 g/h) sont additionnés de méthanol hydraté ou non (débit 6 g/h) et portés à 130°C-140°C pendant 3 heures et recyclés dans la cuve estérificatrice.

Le rendement obtenu lactate de méthyle/acide lactique est supérieur à 98%. La pureté optique de l'ester n'est pas altérée par rapport à celle de l'acide utilisé.

## EXEMPLE 2

Dans le même appareillage que celui décrit dans l'exemple 1, on alimente dans la cuve 20 g/h d'acide lactique hydraté (80% d'acide) et 20 g/h d'isopropanol tout en maintenant sa température à 95-130°C. On souffle à la base de la colonne à plateaux profonds 30 g/h de vapeur d'isopropanol à 120-150°C.

On récupère en tête de l'isopropanol humide qui est régénéré de façon classique avec un entraîneur d'eau. En pied de la colonne à plateaux profonds 50 g/h d'un mélange isopropanol acide lactique, polymère lactique et lactate sont récupérés. Ce mélange est rectifié sous pression réduite pour récupérer l'isopropanol puis le lactate d'isopropyle (23 g/h) (E = 95°C sous 6600 Pa (50 Torr). Les polymères lactiques et l'acide lactique (débit 32 g/h) sont additionnés d'isopropanol hydraté ou non (débit 10 g/h) et portés à 130-140°C pendant 3 heures et recyclés dans la cuve estérificatrice.

Le rendement obtenu lactate d'isopropyle/acide lactique est de 98%. La pureté optique de l'ester n'est pas altérée par rapport à celle de l'acide.

## EXEMPLE 3

Dans l'exemple suivant les parts sont des parts pondérales.

On utilise un ballon tricol en verre surmonté d'une colonne Vigueux avec condenseur et tirage. Le ballon est chauffé électriquement avec régulation de température ; on peut y alimenter avec une pompe péristaltique du cyclohexane en continu.

a) On charge :
— 2 parts d'acide lactique à 81% (2 moles)
— 3 parts d'éthylhexanol

On maintient 120°C dans le ballon pendant 3 h, tout en soufflant 6 parts de cyclohexane. L'acidité du bain passe de 4,3 à 1 équ. d'acide/kg. Le cyclohexane condensé a entraîné :
● 0,6 part d'eau b) On distille le contenu du ballon "in situ", sous vide progressif : le reste de cyclohexane, puis l'éthylhexanol, puis le lactate d'éthylhexyle. Celui-ci passe à 96-100° sous 200 Pa (1,5 Torr). On arrête à 120° en pied de cuve.

Il reste :
● 1 part de résidu
(mélange acide lactique - polylactique et lactate-polylactate).

L'analyse sur les fractions montre que l'on a récupéré :
● 1,2 part d'éthylhexanol
● 2,2 parts de lactate d'éthylhexyl. c) Le résidu est chargé dans un petit autoclave en inox de 500 ml avec :
● 0,2 part d'eau

On chauffe sous agitation 3 h à 135°C. L'acidité passe de 1,1 à 3,6 équ.d'acide/kg.

d) Avec le résidu hydrolysé, on charge dans le ballon de réaction :
● 2 parts d'acide lactique,
● 3 parts d'éthylhexanol.

On chauffe comme en a) en soufflant le cyclohexane. En 3 h, l'acidité passe de 3 à 0,8 équ./kg et l'on condense :
● 0,7 part d'eau e) En distillant comme en b) on récupère :
● 1,2 part d'éthylhexanol,
● 3,1 parts de lactate d'éthylhexyle.

Il reste 1,2 part de résidu.

f) L'analyse (saponification et chromato) montre que le résidu contient 80% d'acide lactique potentiel (donc récupérable par les hydrolyses suivantes).

Pour 4 moles d'acide alimenté, on a donc fabriqué 2,75 mole de lactate d'éthylhexyle et il reste 1,10 mole d'acide lactique.

Le rendement chimique est donc voisin de 96%.

## EXEMPLE 4

On utilise le même appareillage et le même mode opératoire que d ans l'exemple n°3.

a) On charge :
● 2 parts d'acide lactique à 81% (2 moles),
● 3,5 parts d'éthanediol.

On maintient 120°C pendant 4 h en soufflant le cyclohexane.

Celui-ci entraîne :
● 0,7 part d'eau

L'acidité du bain passe de 3 à 0,9 équ. d'acide/kg.

b) On distille comme en b) de l'exemple n° 3. Le monolactated'éthanediol passe à 120-125° sous 120 Pascal (1 Torr).

On récupère dans les fractions :
● 2,7 parts d'éthanediol,
● 1,1 part de monolactate d'éthanediol.

Il reste une part de résidus lourds constitués de dilactate, polylactate et d'acides polylactiques (au-dessus de 135° en pied).

c) Ce résidu est hydrolysé 3 h à 135°C en autoclave avec 0,2 part d'eau.

L'acidité passe de 0,7 à 3,90 équ. d'acide/kg.

d) L'hydrolysat est ajouté à :
● 3 parts d'acide lactique et
● 2 parts d'éthanediol.

En 5 h d'estérification à 120°C, l'acidité passe de 4,3 à 1 équ. d'acide/kg.

Le cyclohexane a entraîné :
● 1 part d'eau

e) On distille comme en b). On récupère :
● 1,3 part d'éthanediol,
● 1,9 part de monolactate d'éthanediol.

Il reste un résidu riche en dilactate de 2 parts.

f) L'analyse montre que ce résidu contient 79% d'acide lactique potentiel. Pour 5 moles d'acide lactique mis en oeuvre, on a donc récupéré 3 moles de monolactate d'éthanediol. Le rendement est donc voisin de 97%.

N.B. : il y a un intérêt à opérer avec un grand excès d'éthanediol, comme dans la première opération, sinon on produit beaucoup de diester à recycler et hydrolyser comme les polylactates. Ce diester est d'ailleurs difficile à séparer du monoester par distillation.

## Revendications

1) Procédé de préparation de lactates d'alkyle optiquement purs par condensation d'un énantiomère de l'acide lactique (A) avec un alcool (B) contenant 1 à 12 atomes de carbone caractérisé en ce que :
— dans au moins une enceinte on effectue la condensation de l'acide lactique (A) avec un excès d'alcool (B) en l'absence de catalyseur à une température inférieure à 140°C, en ce qu'on élimine au moyen d'un entrainement à l'alcool ou au moyen d'un entraîneur l'excès d'alcool et l'eau produite par la réaction d'estérification mais pas l'ester,

— dans au moins une enceinte distincte on élimine le reste de l'alcool et on sépare l'ester par distillation sous pression réduite,

— dans une dernière enceinte on traite le résidu obtenu après distillation de l'ester par un mélange eau alcool et on introduit le mélange issu dudit traitement dans l'enceinte d'estérification.

2) Procédé selon la revendication 1 caractérisé en ce que l'on utilise un rapport molaire de l'alcool à l'acide lactique compris entre 1,3 et 4.

3) Procédé selon la revendication 1 caractérisé en ce que l'alcool est choisi parmi le méthanol, l'éthanol, l'isopropanol, l'éthylhexanol, l'éthanediol.

4) Procédé selon la revendication 1 caractérisé en ce que l'entraineur est un composé formant un azéotrope avec l'eau.

5) Procédé selon la revendication 4 caractérisé en ce que l'entraineur est choisi parmi le toluène, le benzène, le xylène, l'oxyde d'isopropyle, l'oxyde de butyle, le méthyl tertiobutyléther, le cyclohexane.

6) Procédé selon la revendication 4 caractérisé en ce que l'entraineur est le cyclohexane.

7) Procédé selon la revendication 1 caractérisé en ce que dans la dernière enceinte on traite le résidu par le mélange eau-alcool, d'estérification à une température comprise entre 100 et 160°C et de préférence à environ 130°C.


**Patentansprüche**

1. Verfahren zur Herstellung von optisch reinen Alkyllactaten durch Kondensation eines Milchsäure-Enantiomeren (A) mit einem Alkohol (B), der 1 bis 12 Kohlenstoffatome enthält, dadurch gekennzeichnet, daß man

— in mindestens einem geschlossenen Raum die Kondensation der Milchsäure (A) mit einem Überschuß an Alkohol (B) in Abwesenheit eines Katalysators bei einer Temperatur unterhalb 140°C durchführt, daß man mit einem Alkohol-Schleppmittel oder einem Schleppmittel den überschüssigen Alkohol und das bei der Veresterungsreaktion gebildete Wasser, jedoch nicht den Ester, entfernt,

— in mindestens einem weiteren geschlossenen Raum den restlichen Alkohol abzieht und den Ester durch Destillation unter vermindertem Druck abtrennt,

— in einem letzten geschlossenen Raum den nach Abdestillation des Esters erhaltenen Rückstand mit einem Gemisch aus Wasser und Alkohol behandelt und das bei dieser Behandlung erhaltene Gemisch in den Raum für die Veresterung einbringt.

2. Verfahren nach Anspruch 1, dadurch gekennzeichnet, daß man ein Mol-Verhältnis von Alkohol zu Milchsäure im Bereich von 1,3 bis 4 einhält.

3. Verfahren nach Anspruch 1, dadurch gekennzeichnet, daß man den Alkohol unter Methanol, Ethanol, Isopropanol, Ethylhexanol und Ethandiol auswählt.

4. Verfahren nach Anspruch 1, dadurch gekennzeichnet, daß das Schleppmittel eine Verbindung ist, die mit Wasser ein Azeotrop bildet.

5. Verfahren nach Anspruch 4, dadurch gekennzeichnet, daß man das Schleppmittel unter Toluol, Benzol, Xylol, Isopropyloxid, Butyloxid, Methyl-tert.-butylether und Cyclohexan auswählt.

6. Verfahren nach Anspruch 4, dadurch gekennzeichnet, daß das Schleppmittel Cyclohexan ist.

7. Verfahren nach Anspruch 1, dadurch gekennzeichnet, daß man in dem letzten geschlossenen Raum den Rückstand mit dem bei der Veresterung erhaltenen Gemisch aus Wasser und Alkohol bei einer Temperatur im Bereich von 100 bis 160°C, vorzugsweise von etwa 130°C, behandelt.


**Claims**

1. Process for the preparation of optically pure alkyl lactates by condensation of an enantiomer of lactic acid (A) with an alcohol (B) containing 1 to 12 carbon atoms, characterized in that :

— the condensation of lactic acid (A) with an excess of alcohol (B) is carried out in at least one enclosure in the absence of catalyst at a temperature below 140°C, in that the excess of alcohol and the water produced by the esterification reaction, but not the ester, are removed by means of an entrainment using alcohol or by means of an entrainer,

— the remainder of the alcohol is removed in at least one separate enclosure and the ester is isolated by distillation under reduced pressure,

— the residue obtained after distillation of the ester is treated in a last enclosure with a water alcohol mixture, and the mixture originating from the said treatment is introduced into the esterification enclosure.

2. Process according to Claim 1, characterized in that a molar ratio of alcohol to lactic acid of between 1.3 and 4 is employed.

3. Process according to Claim 1, characterized in that the alcohol is chosen from methanol, ethanol, isopropanol, ethylhexanol and ethanediol.

4. Process according to Claim 1, characterized in that the entrainer is a compound forming an azeotrope with water.

5. Process according to Claim 4, characterized in that the entrainer is chosen from toluene, benzene, xylene, isopropyl ether, butyl ether, methyl tert-butyl ether and cyclohexane.

6. Process according to Claim 4, characterized in that the entrainer is cyclohexane.

7. Process according to Claim 1, characterized in that in the last enclosure the residue is treated with the water-alcohol mixture from esterification at a temperature of between 100 and 160°C and preferably at about 130°C.